# EUROPEAN PATENT APPLICATION

(11) **EP 4 632 058 A1**
(43) Date of publication of application: **15.10.2025**
(21) Application number: 24169496.7
(22) Date of filing: 10.04.2024
(51) Int. Cl.: C12M 1/00, C12M 1/26

(54) **METHOD OF OPERATING A PROCESS ARRANGEMENT, IN PARTICULAR BIOPROCESS ARRANGEMENT**

(71) Applicant: Sartorius Stedim Biotech GmbH, 37079 Göttingen (DE)
(72) Inventor: Saballus, Martin, 37079 Göttingen (DE); Kruse, Thomas, 37079 Göttingen (DE); Kampmann, Markus, 37079 Göttingen (DE)
(74) Representative: Gottschald Patentanwälte Partnerschaft mbB

(57) **Abstract**

The invention relates to a Method of operating a process arrangement (1), in particular bioprocess arrangement, wherein the method comprises a separation of two phases of an aqueous-two-phase system (2). It is proposed that the process arrangement (1) comprises a fluidized bed centrifuge (3), that the fluidized bed centrifuge (3) is used for the separation of the two phases of the aqueous-two-phase system (2), that the aqueous-two-phase system (2) is loaded into a rotating chamber (4) of the fluidized bed centrifuge (3) as a fluid flow, that due to the rotation of the chamber (4) the aqueous-two-phase system (2) is centrifuged such that a first phase (7) of the two phases is separated from a second phase (8) of the two phases, and, that due to the fluid flow during the rotation of the chamber (4), the first phase (7) remains in the chamber (4) while the second phase (8) leaves the chamber (4), such that the two phases are separated.

## Description

The present invention relates to a method of operating a process arrangement, in particular bioprocess arrangement, according to the general part of claim 1, to a tube set for use in the method according to claim 19 and to a process arrangement adapted to be used in the method according to claim 20.

In the field of production of bioproducts like viral vectors using cells in bioreactors, lately, efficiency is increasingly becoming a main focus for process design. Extracting viral vectors from a cell broth with lysed cells is particularly challenging because the viral vectors typically have a small size of below 5 µm and are in the same size range as other components, in particular cell debris, present in the cell broth.

It is known from WO 2014/135420 A1 that aqueous-two-phase systems (ATPS) can be used to extract a bioproduct from a cell broth. It is also described that the separation of the phases of the ATPS can be done through mixer settlers or centrifugal extractors. Centrifugal extractors allow a continuous separation, however, a centrifugal extractor needs adjustment of its weir if the relative volumes of the phases change, which is generally an unwanted work step, and particularly problematic, if the relative phase volumes change frequently.

WO 2022/157365 describes that centrifuges can be used to separate two phases of an aqueous-two-phase system and that an electrical conductivity can be measured to detect the different phases. It is then proposed not to use a centrifuge.

It is a challenge to find a way of separating two phases of an aqueous-two-phase system which can be integrated into a process, in particular bioprocess, with low complexity.

The invention is based on the problem of improving the known methods such that a further optimization regarding the named challenge is reached.

The above-noted object is solved by the features of the characterizing part of claim 1.

Fluidized bed centrifuges have been investigated for several use cases and with each use case, the availability of fluidized bed centrifuges increases, making it more interesting to find further use cases. Fluidized bed centrifugation systems enable mild separation of solid particles, such as mammalian cells with diameters of approximately 10 - 100 µm, from its supernatant. However, due to limited g-force of currently max. 3,000 g, small particles such as cell debris or bacteria cells, which are typically below 5 µm in diameter, can hardly be separated with those systems. As one result, fluidized bed clarification of adenoviral vectors and adeno-associated viral vectors from lysed cell broth with a majority of < 5 µm cell debris is not efficient.

The main realization of the present invention is that it is possible to use a process arrangement comprising a fluidized bed centrifuge to separate two phases of an aqueous-two-phase system.

In detail, it is proposed that the process arrangement comprises a fluidized bed centrifuge, that the fluidized bed centrifuge is used for the separation of the two phases of the aqueous-two-phase system, that the aqueous-two-phase system is loaded into a rotating chamber of the fluidized bed centrifuge as a fluid flow, that due to the rotation of the chamber the aqueous-two-phase system is centrifuged such that a first phase of the two phases is separated from a second phase of the two phases, and, that due to the fluid flow during the rotation of the chamber, the first phase remains in the chamber while the second phase leaves the chamber, such that the two phases are separated. The phases are separated due to a density difference between the phases, as is generally the principle of centrifugation.

Using a fluidized bed centrifuge to separate two phases of an aqueous-two-phase system has the advantage that any relative volumes of the phases can be handled by adjusting the filling volume of the chamber and therefore with control measures alone. One phase, usually the heavy phase and an interphase of lysed cells, if present, accumulate inside the chamber while the light phase is flushed out. At some point, the chamber is "full" and the interphase, if present, starts leaving the chamber. If no interphase is present, it is directly the heavy phase starting to leave the chamber. It is possible to detect this change via a camera looking at the chamber or via a conductivity sensor located at a tube behind the chamber. The filling volume can also be calculated as chamber filling volume = volume of chamber divided by (1 - relative volume of target phase). The relative volume is the volume relative to the complete volume of the aqueous-two-phase system. If the aqueous-two-phase system e.g. contains 50 % light phase, 7 % interphase and 43 % heavy phase, said percentages are the respective relative volumes. All percentages herein are volume-percentages.

Claims 2 and 3 relate to preferred use-cases of separating the ATPS which may contain a bioproduct, in particular a small bioproduct which otherwise would be difficult or impossible to harvest via a fluidized bed centrifuge. The proposed method therefore enables a further use-case of a fluidized bed centrifuge.

The ATPS can be used to selectively extract the bioproduct into one of the phases, which is termed the "target phase". However, typically, not all bioproduct will be extracted into the target phase, but some bioproduct, termed "residue" here, among other similar residues, may also remain in the other phase or in an interphase, if present. Depending on the composition of the ATPS, other impurities present in the cell broth, for example DNA, will predominantly be enriched in the other phase or in the interphase, if present. This way, a selective purification of the bioproduct in the target phase is achieved.

Claim 4 describes a preferred embodiment of the process for mixing cell broth containing the bioproduct with the ATPS. This may be done inline shortly before the fluidized bed centrifuge to prevent precipitation. The time needed for the bioproduct to locate itself in the target phase is very low.

Claim 5 describes the mechanism used to separate an ATPS with an interphase.

One embodiment according to claim 6 relates to a conductivity sensor in a tube set connected to the chamber. This conductivity sensor allows detecting when a different phase started leaving the chamber and then switching a subsequent valve or the like to separate the phases.

Preferred embodiments according to claims 7 and 8 describe cycles of loading and discharging the chamber and preferably priming the chamber which may be alternatives or performed one after another. Claim 9 relates to preferred sensors for detecting a loading state of the chamber. As mentioned, a conductivity sensor can be readily used to detect a change in phase composition in a tube. Claims 10 and 11 describe how the described cycles can be used when the bioproduct is located in the light phase (claim 10) or in the heavy phase (claim 11).

Depending on the phase containing the bioproduct (target phase), it is proposed in some embodiments to separate the interphase from the target phase and the other ATPS phase with the fluidized bed centrifuge and not in the tube set behind it (claims 10 and 11). A conductivity sensor may be used to separate, to some extent, the not-target phase and the interphase in the tube set and/or detect a starting discharge of the target phase (i.e. a full chamber). The conductivity sensor senses a change in conductivity which indicates that a different phase is present in a tube or the like. As target phase and interphase are discharged one after the other, the transition can be sensed. An optical sensing of phase transitions inside the chamber is also possible and particularly well suited to detect the transition between the interphase and the light phase.

Notably, one embodiment of claim 12 relates to a possibility of increasing the gained bioproduct by flushing with a replacement liquid before discharging. In one embodiment, the chamber is loaded with ATPS, the light phase with the bioproduct being flushed out while the heavy phase and preferably interphase build up in the chamber. If the chamber is full, loading more ATPS would lead to discharging the interphase (or heavy phase). However, at this point, some light phase with bioproduct is still in the tube set before a harvest vessel or the like. Switching to discharging now would discharge this bioproduct. It is possible however to flush the tube set by loading the chamber further with a replacement liquid, e.g. a liquid having the composition of the light phase without bioproduct. In this way, the chamber is not building up further heavy phase/interphase. The same is true for the heavy phase with a fluid flow in the backward direction.

An interesting embodiment is subject of claim 13. To further increase the bioproduct yield and/or reduce the footprint, the bioreactor (or any other vessel) may be connected to an exit port of the tube set, another exit port being used for harvest, and/or a media port of the tube set. The ATPS added to the bioreactor after a bioprocess in the bioreactor can then be used for priming the chamber with the output of the chamber being fed back to the bioreactor. Also, the phases not containing the bioproduct, except for residues, can be fed back to the bioreactor and therefore reenter the circle. This allows harvesting at least some of the residue of bioproduct in the wrong phases.

Claims 14 to 16 further expand this embodiment by describing how this cycle (batch) is repeated (claim 14), how the relative phase volumes in the bioreactor change and how this can be dealt with by replenishing the depleted phase either continuously or preferably starting at some point (claim 15) and how the chamber filling volume can be adapted, in a calculated manner or through the use of the camera or the conductivity sensor, to deal with the change of the relative phase volumes (claim 16).

It has also been found that the ATPS or one of the phases can be used to lyse cells and release the bioproduct, thereby not needing a separate lysis step (claim 17 and 18). The mixing and lysing can be done in the bioreactor as an addition to the embodiments of claims 13 to 16 or applied to a bleed of the bioreactor (claim 18).

Another teaching according to claim 19, which is of equal importance, relates to a tube set for use in the proposed method.

All explanations given with regard to the proposed method are fully applicable.

Another teaching according to claim 20, which is of equal importance, relates to a process arrangement adapted to be used in the proposed method.

All explanations given with regard to the proposed method and the proposed tube set are fully applicable.

In the following, embodiments of the invention are explained with respect to the drawing. The drawing shows in
- Fig. 1,: an overview of a bioprocess arrangement with a fluidized bed centrifuge, a bioreactor, a media vessel and a waste vessel,
- Fig. 2,: the working principle of the ATPS separation via a fluidized bed centrifuge,
- Fig. 3,: a first scenario, here with the bioproduct located in the light phase,
- Fig. 4,: a second scenario in which the waste port and the media port are connected to the bioreactor, here with the bioproduct located in the light phase,
- Fig. 5,: the first scenario, here with the bioproduct located in the heavy phase and
- Fig. 6,: the second scenario, here with the bioproduct located in the heavy phase.

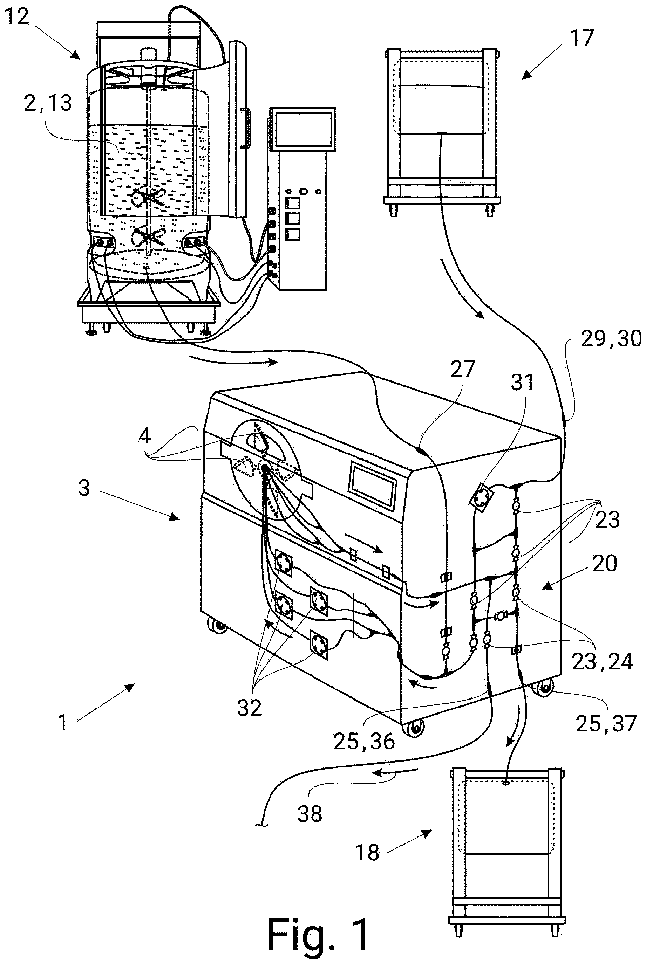

Proposed is a method of operating a process arrangement 1, in particular bioprocess arrangement. Fig. 1 shows a bioprocess arrangement. The method comprises a separation of two phases of an aqueous-two-phase system 2.

Proposed is that the process arrangement 1 comprises a fluidized bed centrifuge 3 and that the fluidized bed centrifuge 3 is used for the separation of the two phases of the aqueous-two-phase system 2.

The term "fluidized bed centrifuge" 3 refers to a type of counter-flow centrifuge known to the person skilled in the art. The term "fluidized bed" in "fluidized bed centrifuge" 3 originates from a process performable with a fluidized bed centrifuge 3 in which a fluidized bed is formed inside the chamber 4 of the fluidized bed centrifuge 3 by loading a solid-liquid suspension into the chamber 4 as a fluid flow and balancing the forces inside the chamber 4 such that a fluidized bed is formed. The fluidized bed can then be discharged, leading to a sort of batch process for separating liquid and solid. It is noted that usually a fluidized bed centrifuge 3 is said to be continuous if looking at an integration into a process and not individual discharges. It should be understood that the term "fluidized bed centrifuge" 3 is used here to refer to the device that is generally able to be used as a fluidized bed centrifuge 3, and therefore also called "fluidized bed centrifuge" 3, but in the present method is not used in the usual manner.

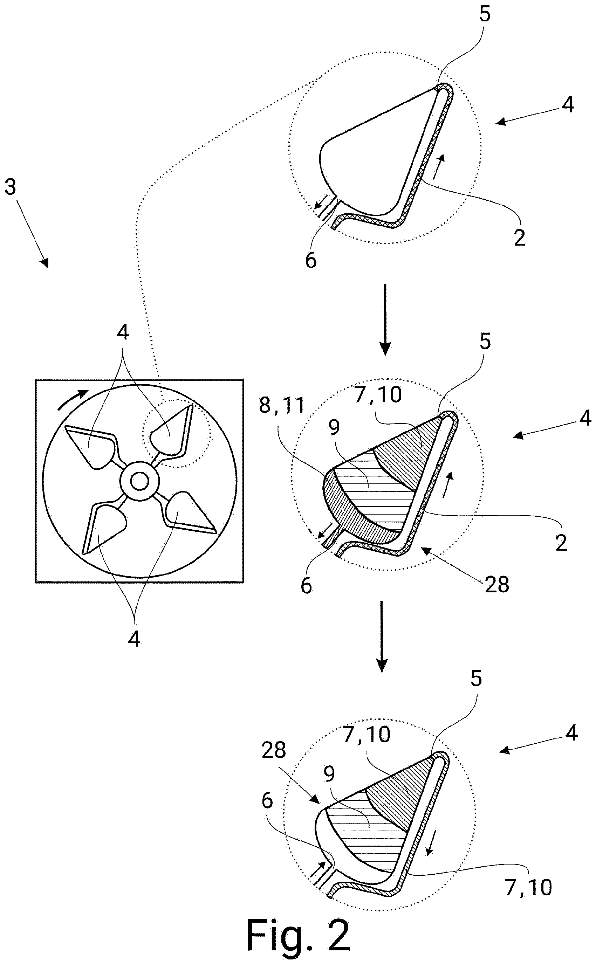

To achieve the separation of the two phases of the aqueous-two-phase system 2, the aqueous-two-phase system 2 is loaded into a rotating chamber 4 of the fluidized bed centrifuge 3 as a fluid flow. Fig. 2 shows a chamber 4 of the fluidized bed centrifuge 3. Generally and preferably in the present case, the fluidized bed centrifuge 3 operates via the counter-flow principle. The fluid flow flows in a forward direction which is a direction opposed to the force generated by the rotation of the chamber 4. Fig. 2 shows three views of the chamber 4. In the top two views the fluid flow is directed in the forward direction as shown by the arrows.

Here and preferably the chamber 4 comprises, in particular exactly, one chamber inlet 5 and, in particular exactly, one chamber outlet 6. Here and preferably, the fluidized bed centrifuge 3 comprises at least two, preferably at least or exactly four, chambers 4.

Due to the rotation of the chamber 4 the aqueous-two-phase system 2 is centrifuged such that a first phase 7 of the two phases is separated from a second phase 8 of the two phases. For this functionality, the fluidized bed centrifuge 3 behaves like any centrifuge. The two phases of the aqueous-two-phase system 2 are in a mostly mixed state when entering the chamber 4 or more precisely the rotating tube leading to the chamber 4. Due to the high g-forces, the two phases separate very quickly such that, as with a usual centrifuge, the phases separate inside the chamber 4 and, to some extent, the tube leading to the chamber 4.

Due to the fluid flow during the rotation of the chamber 4, the first phase 7 remains in the chamber 4 while the second phase 8 leaves the chamber 4, such that the two phases are separated. At the chamber inlet 5, the fluid flow consists of the aqueous-two-phase system 2 in a mixed state. Through the chamber 4, the first phase 7 separates from the second phase 8 and fills up the chamber 4 from the chamber inlet 5 towards the chamber outlet 6. The fluid flow at the chamber outlet 6 mostly consists of the second phase 8 until the chamber 4 is filled with the first phase 7 and possibly an interphase 9 yet to be explained. The fluid flow is set low enough such that the g-forces from the rotation are high enough to mostly separate the phases inside the chamber 4. That is, for low rotational speed and high fluid flow, at some point, the mechanism would not work properly.

The topmost view of the chamber 4 in Fig. 2 shows an empty, but primed, chamber 4. The middle view shows an almost full chamber 4. Here, the first phase 7, here and preferably the heavy phase 10 of the aqueous-two-phase system 2, is filling the chamber 4 from the chamber inlet 5. A yet to be explained and optional interphase 9 is present in the middle of the chamber 4 due to the same principle. The second phase 8, here and preferably the light phase 11, is driven out of the chamber 4 by the fluid flow. As long as the chamber 4 is not filled with the heavy phase 10 and the interphase 9, some light phase 11 remains in the chamber 4.

The bottom view in Fig. 2 shows the backward operation in which the content of the chamber 4 is discharged by reversing the fluid flow, indicated by arrows. This discharge may be done phase by phase with a low fluid flow or all at once with a high fluid flow.

As a fluidized bed centrifuge 3 is used, the two phases are separated in a batch operation and not continuously as, preferably, forward and backward direction are alternated. More precisely, in the preferred embodiments, the second phase 8 is gathered mostly continuously while the fluid flow is active while the first phase 7 is discharged regularly from the chamber 4. An example of a fluidized bed centrifuge 3 is the Sartorius kSep.

As a side note, it should be understood that when saying that (only) the light phase 11 leaves the chamber 4, the light phase 11 is not 100% pure and contains to some percent, e.g. 3 %, heavy phase 10 and interphase 9. The wording is in line with usual wordings in the present field. As the same is true for the other phases, not all of the light phase 11 leaves the chamber 4, even if the chamber 4 is full. The same is true for other similar wordings, e.g. that the bioproduct is located in one phase. Most of the bioproduct is then located in said phase but naturally not all of it. References to phases therefore mean liquids generally composed of that phase with normal residues of other phases.

The term "bioproduct" in general refers to a product produced by the cells cultivated. Cultivation of cells may currently be used for the production of biopharmaceuticals, in particular proteins, such as human insulin or monoclonal antibodies but also antibody-drug conjugates or the like. The bioproduct may as well be a viral vector, preferably an adenoviral or adeno-associated viral vector or a lentiviral vector that is purified and may for example be used for applications in the emerging field of cell and gene therapy.

Turning from the basic principle back to the preferred use case, Fig. 1 shows that the bioprocess arrangement here and preferably comprises a bioreactor 12. In the bioreactor 12, a bioproduct is produced by cells, preferably mammalian cells and a cell broth 13 is obtained. The cell broth 13 comprises the cells and the bioproduct. In particular to harvest the bioproduct, the cell broth 13 is transferred from the bioreactor 12 to the fluidized bed centrifuge 3. This transfer may be done directly or indirectly through a holding vessel or the like.

The cell broth 13 is mixed with two phase-forming components 14, in particular phosphate salt 15 and polyethylene glycol (PEG 16), to form the aqueous-two-phase system 2. If, as is preferred, the bioproduct is present in the cell broth 13 outside the cells, possibly because the cells are lysed, the bioproduct transfers into one of the two phases. Here and preferably, the light phase 11 is PEG-rich, the heavy phase 10 is salt-rich and due to the biomass, an interphase 9 containing the lysed cells forms between the light phase 11 and the heavy phase 10.

Other types of ATPS may be used, preferably polymer-polymer systems, in particular PEG 16 and dextran, or polymer-salt systems, in particular PEG 16 and phosphate or sulfate or citrate or alcohol-salt ATPS or micellar ATPS or ionic based ATPS.

According to one embodiment it is proposed that the bioproduct has a size of at most 5 µm, preferably at most 1 µm, more preferably at most 0,5 µm. The bioproduct may be a viral vector. Preferably, the bioproduct is an adenoviral vector or an adeno-associated viral vector or a lentiviral vector or a virus-like particle or a recombinant protein or a nucleic acid or a monoclonal antibody or an antibody-drug conjugate.

Generally, the bioproduct may be enriched in the light phase 11 or the heavy phase 10. For a viral vector, usually and preferably, the viral vector is enriched in the light phase 11, while impurities like DNA and host cell proteins are enriched in the interphase 9 and/or the heavy phase 10. This is particularly advantageous as then no nuclease treatment may be needed.

The interphase 9 formation is driven by molecular interactions (volume exclusion effects), not only by g-force, thus sub-micrometer cell debris are collected in this fraction and a highly efficient capture of lysed cells inside the chamber 4 is possible. Therefore, only a small subsequent in-line filter for the light phase 11 is needed.

Further, the PEG 16 inside the light phase 11 could be favorable to maintain the viral vector activity during freezing or short-term storage reducing the need for cryoadditives.

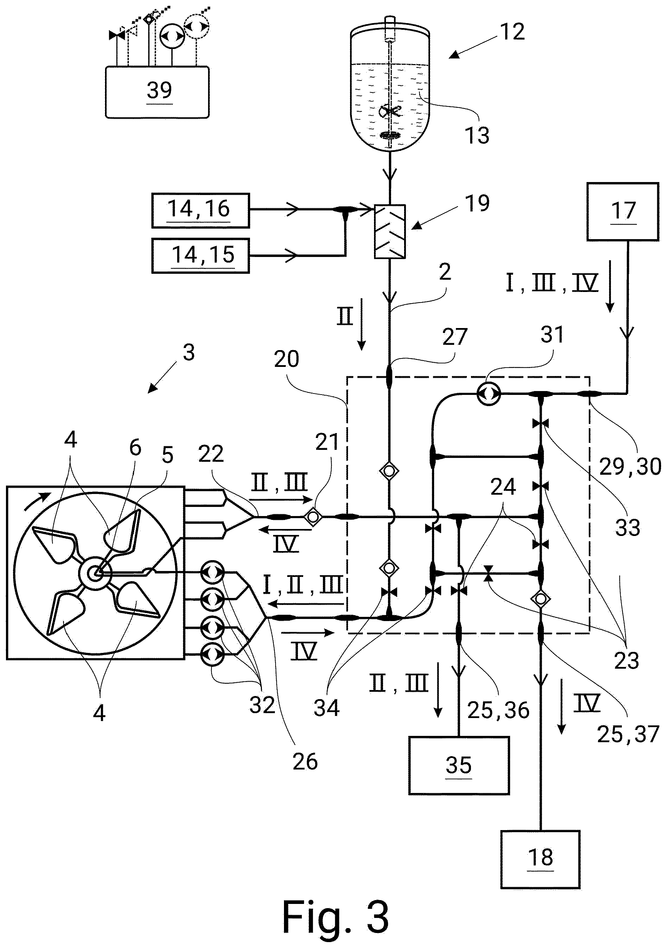

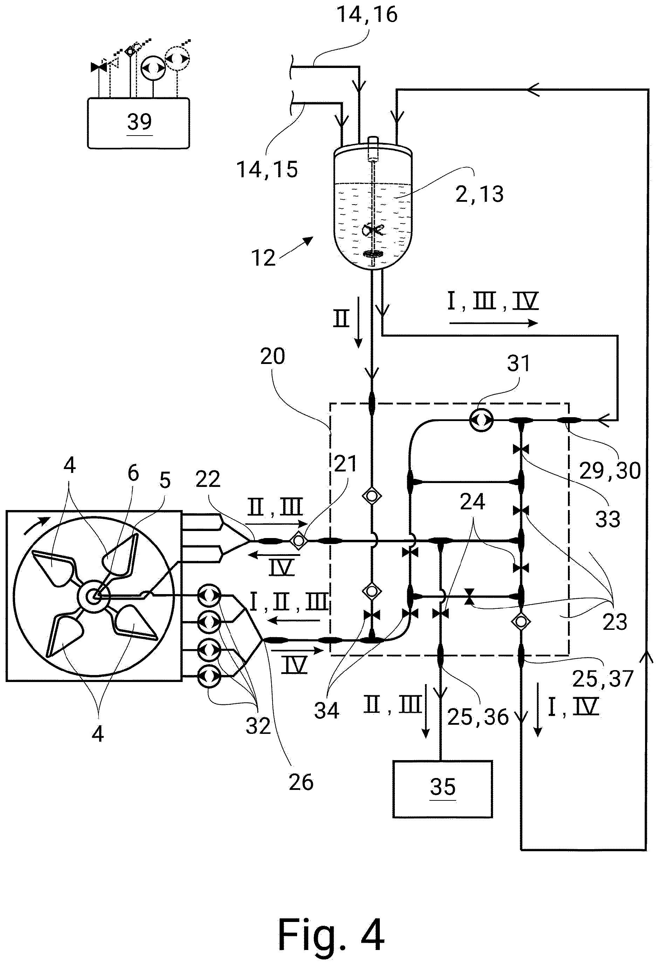

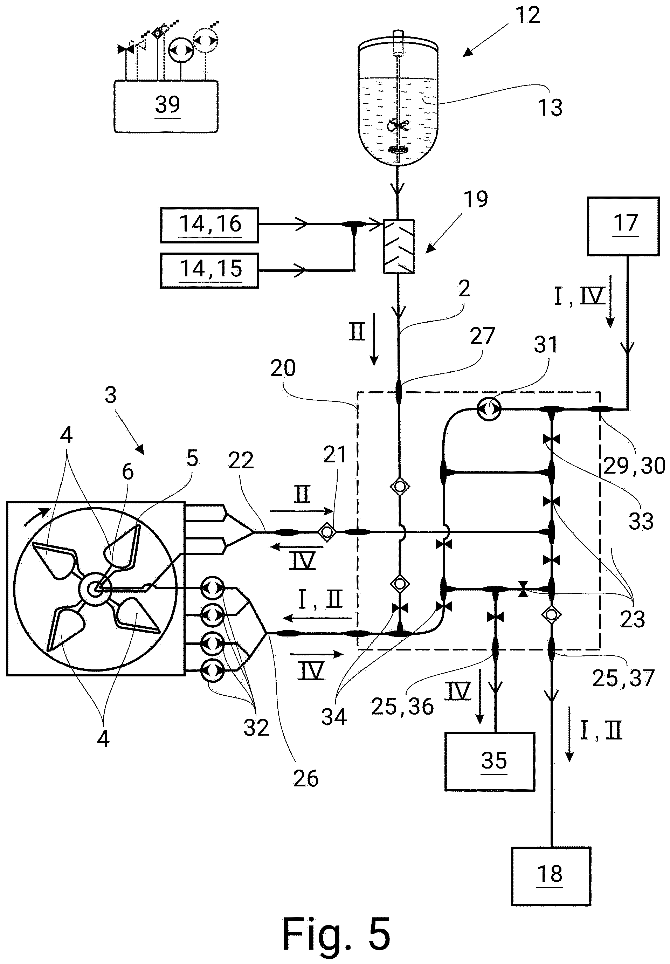

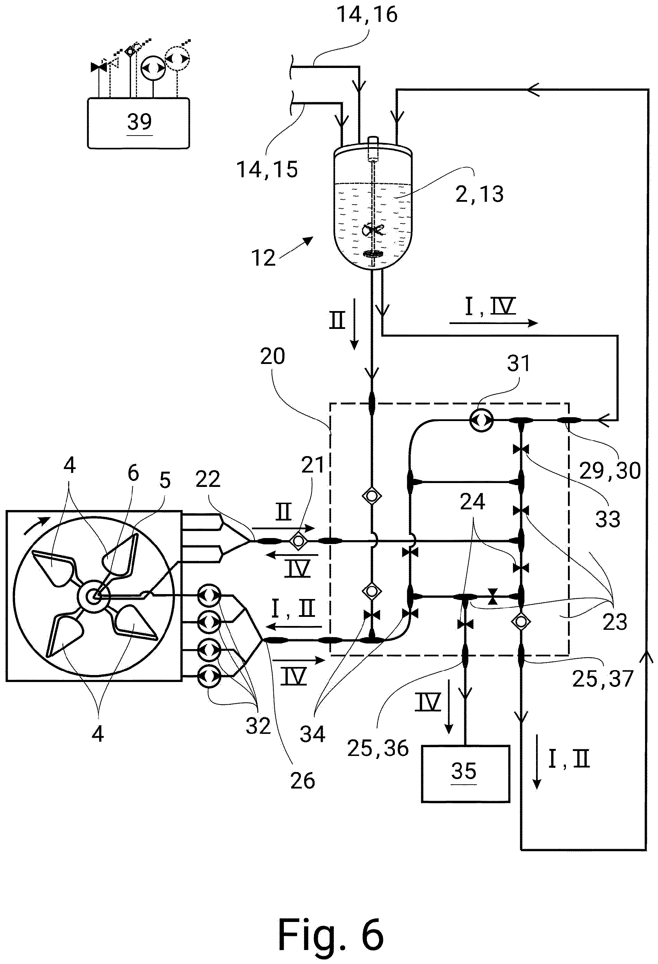

Figs. 3 to 6 show different preferred embodiments of how the separation of two phases of an ATPS can be used to gain a bioproduct. Figs. 3 and 5 show a first scenario and Figs. 4 and 6 show a second scenario. In the first scenario, a separate media vessel 17 for a replacement liquid is used and a discharge of the chamber 4 is transferred into a waste vessel 18. The first scenario therefore corresponds to the process arrangement 1 shown in Fig. 1, though Fig. 3 shows more details. In the second scenario, the phase-forming components forming the aqueous-two-phase system 2 are added directly to the cell broth 13 in the bioreactor 12 to form the aqueous-two-phase system 2. The ATPS is then used to prime the chamber 4 and the discharge of the chamber 4 is returned to the bioreactor 12. This scenario needs less media and allows harvesting more bioproduct by mixing the discharge, which contains residues of the bioproduct, back into the bioreactor 12 and therefore back into the harvest cycle. This will lead to a depletion of the phase with the bioproduct in the bioreactor 12. At some point, the phase with the bioproduct can be replenished which allows more harvest cycles of the fluidized bed centrifuge 3 and a higher yield of the bioproduct. Notably, a fluidized bed centrifuge 3 can deal very well with the changes in relative phase volume.

In Figs. 3 and 4 the bioproduct is located in the light phase 11 and in Figs. 5 and 6 the bioproduct is located in the heavy phase 10.

The shown scenarios are merely exemplary combinations of different features and some possible changes to the scenarios will also be described. Most described features can be realized independently of the other features of the respective scenario.

According to one embodiment it is proposed that at least one of the two phase-forming components 14, preferably both phase-forming components, are added to the cell broth 13 inline while the cell broth 13 is flowing between the bioreactor 12 and the fluidized bed centrifuge 3. This is realized in the first scenario in Figs. 3 and 5 but not necessary for this scenario. Preferably, the cell broth 13 and the two phase-forming components 14 are mixed inline by a static mixer 19. Here and preferably, the cell broth 13 flows directly from the bioreactor 12 to the fluidized bed centrifuge 3.

It is preferably the case that after the two phase-forming components 14 have been added to the cell broth 13 thereby creating the aqueous-two-phase system 2, the aqueous-two-phase system 2 is transferred into the chamber 4 of the fluidized bed centrifuge 3 after at least 2 seconds, preferably at least 5 seconds, and/or at most 60 minutes, preferably at most 10 minutes, more preferably at most 2 minutes. This may be the case irrespective of whether the mixing is done inline or not. The named times start running after the second phase-forming component forming the ATPS has been added. Preferably, the same times apply alternatively after the last mixing before the fluidized bed centrifuge 3, e.g. here after the ATPS leaves the static mixer 19.

According to one embodiment it is proposed that inside the centrifuge chamber 4 a light phase 11, in particular a PEG-rich phase, an interphase 9, in particular containing biomass, and a heavy phase 10, in particular a salt-rich phase, of the aqueous-two-phase system 2 are located at different regions of the chamber 4. This mechanism has been explained above in general. The interphase 9 is here and preferably a phase consisting mainly of lysed cells which forms if an ATPS is added to a cell broth 13 and the cells are lysed. Preferably, two of the three phases are retained in the chamber 4 and the third phase is flushed out of the chamber 4. Alternatively, one of the three phases is retained in the chamber 4 and the other two phases are flushed out of the chamber 4.

Before looking at the scenarios in more detail, it may generally be the case that a tube set 20 is connected to, in particular forms a, preferably two-part, single-use unit with, the fluidized bed chamber 4. The tube set 20 may be made out of a single use material. It may come in a pre-mounted form, sterilized and packaged in a sterile manner. It can then be mounted to the fluidized bed centrifuge 3. The tube set 20 may comprise several ports that will be explained later.

Here and preferably, the tube set 20 comprises a conductivity sensor 21. With the conductivity sensor 21, a change between phases at the position of the conductivity sensor 21 can be detected. Here and preferably, the tube set 20 is a concentration-wash-harvest tube set 20 or a harvest-clarification tube set 20 with an added conductivity sensor 21.

Preferably, the conductivity sensor 21 is connected to an output fluid line 22 of the fluidized bed chamber 4, and more preferably, the conductivity sensor 21 is used to separate the two phases by adapting the fluid flow, in particular by reversing the fluid flow, and/or by switching a valve 23 of the tube set 20 connected to the output fluid line 22. The tube set 20 may be mounted to the fluidized bed centrifuge 3 such that one or, as here, two, exit valves 24 can be used to direct the fluid leaving the chamber 4 through the output fluid line 22 to one of at least two exit ports 25 (first scenario).

Alternatively, the conductivity sensor 21 may be connected to an input fluid line 26. For discharging the phases in the chamber 4, the fluid flow may be reversed. The phases are then preferably discharged one after the other. The conductivity sensor 21 may be used to sense the transition between the phases and preferably to switch a valve 23 based on the detection of the transition.

Two scenarios for the use of a fluidized bed centrifuge 3 to separate two phases of an aqueous-two-phase system 2 will now be explained with regard to Figs. 3 to 6. These provide exemplary understanding of the principles that can be used but are not the only possible scenarios.

According to one embodiment building the basis for the shown scenarios it is proposed that a cycle of the fluidized bed centrifuge 3 comprises the following steps in this order:
a) the chamber 4 is loaded with the aqueous-two-phase system 2 as the fluid flow in the forward direction, in particular through an input port 27 of the tube set 20, and the light phase 11 is flushed out of the chamber 4 in the forward direction during the loading while the heavy phase 10 remains in the chamber 4,
b) the loading of step a) is finished, preferably at a time determined based on an optical detection of a predefined loading state of the chamber 4,
c) the heavy phase 10 is flushed out of the chamber 4 in the backward direction.

The input port 27 may be a dedicated port of the tube set 20 which can be connected to different vessels. Here and preferably, the input port 27 is connected to a vessel containing the aqueous-two-phase system 2, in particular the bioreactor 12. From the input port 27 the ATPS flows as the fluid flow through the chamber inlet 5 into the chamber 4. In step a), the heavy phase 10 gradually builds up in the chamber 4 while preferably most of the light phase 11 is flushed out through the chamber outlet 6. The interphase 9 may also remain in the chamber 4 or may be flushed out of the chamber 4. The time for finishing step a) may be determined based on a camera or other optical sensor aimed at the chamber 4 when the interphase 9 or the heavy phase 10 reaches the elutriation boundary 28, here and preferably the widest part of the chamber 4 or the end of the chamber 4 or any other predefined level. The chamber 4 is then preferably full, adding further ATPS would lead to flushing out the interphase 9 or heavy phase 10. In step c), the flow direction is reversed, thereby operating the chamber 4 in backward operation and the heavy phase 10 and possibly the interphase 9 is or are flushed out of the chamber 4 through the chamber inlet 5.

The cycle of the fluidized bed centrifuge 3 may comprise before step a) a step of priming the chamber 4 through a priming port 29. The priming port 29 is also connected to the chamber inlet 5. The priming may be done with the aqueous-two-phase system 2 (Figs. 4 and 6 as will be explained) or a priming liquid, in particular coming from a media vessel 17 (Figs. 3 and 5). The fluid flow in each step is shown with Roman numerals I to IV in Figs. 3 to 6. Numeral I shows the fluid flow during the priming step. II shows the fluid flow during step a), III shows an optional further loading step that will be explained and IV shows the fluid flow in step c).

Alternatively or additionally in a scenario not shown but equally preferred and understandable based on the shown embodiments, it may be the case, that a cycle of the fluidized bed centrifuge 3 comprises the following steps in this order:
d) the chamber 4 is loaded with the aqueous-two-phase system 2 as the fluid flow in the backward direction and the heavy phase 10 is flushed out of the chamber 4 in the backward direction during the loading while the light phase 11 remains in the chamber 4,
e) the loading of step d) is finished, preferably at a time determined based on a detection of a predefined loading state of the chamber 4 by the conductivity sensor 21,
f) the light phase 11 is flushed out of the chamber 4 in the forward direction.

Staying with the definition of input and output used before, the chamber 4 in d) may be loaded through the chamber outlet 6. In d), the interphase 9 may remain in the chamber 4 or be flushed out, too. One or more conductivity sensors 21 may be located at the chamber inlets 5 and may be used to detect the predefined loading state.

As can be seen, it is possible to use the chamber 4 in the opposite direction to the scenarios shown. Depending on the tube set 20 or more generally any fluidical network connected to the chamber inlet 5 and the chamber outlet 6, it is possible to switch valves 23 of the tube set 20 such that the chamber 4 is loaded with the aqueous-two-phase system 2 until the chamber 4 is filled with heavy phase 10 and possibly interphase 9 in one direction of fluid flow and then loaded with the the aqueous-two-phase system 2 until the chamber 4 is filled with light phase 11 and possibly interphase 9 in the other direction and then filled with heavy phase 10 and possibly interphase 9 again, and so on. It is therefore generally preferably the case that one or more conductivity sensors 21 are connected to the chamber inlet 5 and/or to the chamber outlet 6 to detect a predefined loading state of the chamber 4. Additionally and/or alternatively, an optical sensor may be used to detect the predefined loading state of the chamber 4 while the fluid flow is active in the forward and/or backward direction.

According to one embodiment, and shown exemplarily in Figs. 3 and 4, it is proposed that the bioproduct is located in the light phase 11.

It may then be the case that in step a) the interphase 9 remains in the chamber 4. Therefore, during step a) mainly the light phase 11 is flushed out through the chamber outlet 6 and the loading is stopped if the chamber 4 is filled with heavy phase 10 and interphase 9. In step d), the interphase 9 may be flushed out of the chamber 4 with the heavy phase 10 until mainly light phase 11 remains. Here and preferably, the tube set 20 has two exit ports 25. The exit ports 25 may have a preferred use for either harvest or waste, but it may also be possible to use the exit ports 25 differently. Switching between exit ports 25 can then be done based on a measurement of the conductivity sensor 21. Otherwise, preferably, in step c) the interphase 9 is flushed out of the chamber 4 in the backward direction, together with the heavy phase 10 or remains in the chamber 4 together with the light phase 11 in step d) and is flushed out in step f). The interphase 9 can then also be separated by switching a valve 23 as the phases can be flushed out one after another.

When the chamber 4 is fully loaded further addition of ATPS would lead to interphase 9 or heavy phase 10 leaving the chamber 4 (step a)). However, at that point, the tubes of the tube set 20 still contain light phase 11 with bioproduct and reversing the fluid flow would pump this bioproduct backwards instead of harvesting it.

Therefore, preferably and independent of the location of the bioproduct in the heavy phase 10 or light phase 11, the cycle of the fluidized bed centrifuge 3 comprises between steps b) and c) and/or e) and f) a step of further loading the chamber 4 with a replacement liquid as the fluid low. Between steps b) and c) this flushes light phase 11 out of the tube set 20 behind the chamber 4. Between steps e) and f) heavy phase 10 is flushed out instead. By loading the chamber 4 further with liquid that does not build up in the chamber 4, it is possible to flush the tubes behind the chamber 4 "through" the chamber 4, still in forward or backward operation and recover the bioproduct in the tubes. The replacement liquid may have the composition of the target phase but without bioproduct. The same may be true additionally or alternatively for the priming liquid. Therefore, the media vessel 17 shown in Figs. 3 and 5 may contain the replacement liquid being the priming liquid and having the composition of the target phase.

Looking at Fig. 3 now, summarized, in a preferred embodiment the chamber 4 is first primed with priming liquid from the media vessel 17 through the media port 30 of the tube set 20 (numeral I). Fig. 3 shows a media pump 31 which may be bypassed for priming if the priming is done by chamber pumps 32. A bypass valve 33 acts on the tube set 20 accordingly. The priming liquid may be discarded into a waste vessel 18.

After priming, an input switch valve 34 closes the connection to the media vessel 17. Numeral II shows the ATPS coming from the bioreactor 12 being loaded into the chamber 4 (step a)). Closely after or at the same time the exit valves 24 are switched so that the light phase 11 leaving the chamber 4 is transferred into a harvest vessel 35. An initial volume in the tube set 20 behind the chamber 4 with priming liquid can be discharged by switching the exit valves 24 later. This fluid flow is controlled by the chamber pumps 32.

When the chamber 4 is full, the chamber 4 is flushed to move the light phase 11 behind the chamber 4 into the harvest vessel 35. For this, the input switch valve 34 switches while the exit valves 24 stay as they are (numeral III). For better understanding, the same steps have the same numerals in all figures even though this step may be omitted if the bioproduct is in the heavy phase 10 and is therefore not shown in Fig. 5 and 6.

Then, the exit valves 24 are both closed and the fluidized bed centrifuge 3 is operated backwards. Replacement liquid from the media vessel 17 is flushed backwards through the chamber 4 by the media pump 31 and into the waste vessel 18 to remove the heavy phase 10 and the interphase 9 (numeral IV). Afterwards, the cycle can be repeated. The function of the other valves 23 not explicitly named is obvious from the respective fluid flows.

According to one embodiment building the basis of Figs. 5 and 6 it is proposed that the bioproduct is located in the heavy phase 10, that in step a) the interphase 9 is flushed out of the chamber 4. Alternatively or additionally if steps a) to c) and d) to f) are all performed, it may be the case that in step d) the interphase 9 remains in the chamber 4 and in step f) the interphase 9 is flushed out of the chamber 4. The chamber 4 may therefore be loaded until it contains mainly heavy phase 10 in step a) or the heavy phase 10 is flushed out mostly without interphase 9 in step d).

Preferably, in step a), a valve 23, here an exit valve 24, of the tube set 20 is switched based on a measurement of the conductivity sensor 21 to pump the light phase 11 and the interphase 9 through different exit ports 25 of the tube set 20 (not shown, would necessitate a further exit port 25 in the shown embodiments) and/or a starting discharge of the heavy phase 10 in the forward direction is detected via the conductivity sensor 21. If the discharge of the heavy phase 10 is detected in the tube set 20, heavy phase 10 has left the chamber 4 and the chamber 4 is full of heavy phase 10. If the fluid flow is reverted quickly, the heavy phase 10 in the tube set 20 can still be harvested as in the backward operation, the same tube is flushed in the other direction. Alternatively, an optical measurement can be used to detect when the chamber 4 is full. Here, the interphase 9, or rather the fluid leaving the centrifuge chamber 4 after the light phase 11, will contain also a substantial amount of light phase 11 as the fluid flow is still active in the forward direction.

In one embodiment, after step b) and before step c) and/or after step e) and before step f) the flow is stopped and the chamber 4 keeps rotating to improve the phase separation in the chamber 4. This step may be done for example for 10 seconds, here and preferably at least 3 seconds and/or at most 30 seconds.

Equally, for the bioproduct in the light phase 11, in step d), a starting discharge of the heavy phase 10 in the forward direction may be detected via the conductivity sensor 21 and/or a valve 23 of the tube set 20 is switched based on a measurement of the conductivity sensor 21 to pump the heavy phase 10 and the interphase 9 through different ports of the tube set 20.

Fig. 5 therefore shows numerals I for priming from the media vessel 17, II for loading and sending light phase 11 and interphase 9 to the waste vessel 18, and IV for discharging the heavy phase 10 into the harvest vessel 35. The key difference is that the bioproduct is mainly obtained during the forward operation, when the bioproduct is in the light phase 11, but it is mainly obtained during the backward operation, when the bioproduct is in the heavy phase 10.

Generally, it can now be understood that the tube set 20 may comprise an input port 27 for connection to the bioreactor 12, a harvest port 36 for connection to a harvest vessel 35, which harvest port 36 may be connected to the chamber inlet 5 or the chamber outlet 6 depending on the location of the bioproduct in the light phase 11 (Figs. 3 and 4) or the heavy phase 10 (Figs. 5 and 6), a waste port 37 which can be connected to the waste vessel 18 and a media port 30 which may be connected to the media vessel 17. The conductivity sensor 21 may be located behind the chamber outlet 6 in the tube set 20.

According to an embodiment building the basis for Fig. 4 it is proposed that an exit port 25 of the tube set 20 is connected to the vessel, in particular the bioreactor 12, containing the aqueous-two-phase system 2 and the heavy phase 10 and preferably the interphase 9 in step c) is or are discharged into the bioreactor 12. Starting from the description of Fig. 3 and with the bioproduct in the light phase 11, one difference towards Fig. 4 is that instead of a waste vessel 18 the discharge is transferred back to the vessel and subsequently again subjected to the fluidized bed centrifuge 3. This allows residues of bioproduct in the heavy phase 10 or the interphase 9 to move into the light phase 11 in the vessel and then to be harvested in a subsequent cycle. Additionally or alternatively, the light phase 11 and preferably the interphase 9 in step f) is or are discharged into the bioreactor 12 if the bioproduct is in the heavy phase 10.

Alternatively or additionally it may be the case, as shown in Fig. 6, that an exit port 25 of the tube set 20 is connected to a vessel, in particular the bioreactor 12, containing the aqueous-two-phase system 2 and the light phase 11 in step a) or heavy phase 10 in step f) is discharged into the bioreactor 12. This is the same as before except with the bioproduct being in the other phase.

In both embodiments or independently it may be the case that the media port 30 is connected to the vessel, in particular the bioreactor 12, containing the aqueous-two-phase system 2. This allows priming and/or discharging with ATPS and reduces the necessary further media and the necessity of a waste vessel 18.

According to one embodiment it is proposed that one or both phase-forming components are added to the bioreactor 12 or a subsequent vessel, that the cell broth 13 is transferred in batches to the fluidized bed centrifuge 3, that, for processing each batch, the phase containing the bioproduct is separated in the fluidized bed chamber 4 from the interphase 9 and the other phase and the phase containing the bioproduct is transferred to a further processing 38, e.g. by first transferring it into the harvest vessel 35. Preferably, the other phase and the interphase 9 are returned to the bioreactor 12 or the vessel.

In the embodiments where the discharge is returned to the vessel it may be the case that when the relative volume of one phase in the vessel, in particular bioreactor 12, fulfills a criterion, in particular reaches a threshold, the phase-forming component forming the phase containing the bioproduct is replenished in the vessel and further batches are processed. Starting with relative phase volumes of e.g. around 50 % light phase 11 and heavy phase 10 if the light phase 11 contains the bioproduct it may be the case that after reaching a threshold of relative volume, e.g. 10 %, of light phase 11 in the vessel the light phase 11 is replenished continuously to keep the relative amount of light phase 11 around the threshold, here 10 %. The same may be done alternatively for the heavy phase 10. Therefore, preferably, the replenishment is done continuously with a flow rate equal to an average transfer of volume to the further processing 38. Alternatively, the phase-forming component forming the phase containing the bioproduct may be replenished continuously or according to any other strategy.

According to one embodiment it is proposed that depending on the relative volume of one phase in the bioreactor 12 or vessel a chamber 4 filling volume of the fluidized bed centrifuge 3 is adapted between batches.

An experiment was conducted with only ATPS leading to 98 % light phase 11 and 2% heavy phase 10 in the harvest vessel 35 when going from 50 % light phase 11 and 50 % heavy phase 10 in the vessel representing the bioreactor 12 to 90 % heavy phase 10 and 10 % light phase 11 according to the embodiment in Fig. 4. The result of 98 % light phase 11 in the harvest vessel 35 shows that the separation works.

Another preferred embodiment relates to the lysis of the cells. It is preferably the case that the bioproduct is produced inside the cells and that at least one of the phase-forming components lyses the cells to release the bioproduct. Preferably, the cell broth 13 is mixed with a first of the two phase-forming components 14, in particular a salt-rich phase-forming component, preferably wherein the salt is a phosphate salt 15, then the cells are lysed and then the cell broth 13 is mixed with the second of the two phase-forming components 14. A separate step for lysing the cells is therefore no necessary. The mixing can be done inline or in the vessel as explained.

Alternatively, the cell broth 13 may be mixed with both of the phase-forming components and the combination of both phase-forming components may lyse the cells. An interesting observation is that most host cell impurities e.g. DNA are enriched in the heavy phase 10 or the interphase 9 for some ATPS phase-forming components where the bioproduct is solved in the light phase 11, e.g. for PEG 16 and phosphate salt 15.

According to one embodiment it is proposed that a fraction of the cell broth 13 in the bioreactor 12 is transferred to the fluidized bed centrifuge 3, that the two phase-forming components 14 are added to the fraction of the cell broth 13 inline, that the cells are lysed, that the bioproduct is an intracellular bioproduct, in particular a viral vector, that the phase containing the bioproduct is separated in the fluidized bed chamber 4 from the interphase 9 and the other phase, and, that a fraction of the cell broth 13 remaining in the bioreactor 12 is further cultivated.

Another teaching which is of equal importance relates to a tube set 20 for use in the proposed method, wherein the tube set 20 comprises a conductivity sensor 21. Preferably, the tube set 20 is a single-use tube set 20.

All explanations given with regard to the proposed method are fully applicable.

Another teaching which is of equal importance relates to a process arrangement 1 adapted to be used in the proposed method. The process arrangement 1 may comprise a control unit 39 adapted to perform the proposed method.

All explanations given with regard to the proposed method and the proposed tube set 20 are fully applicable.

### Reference numbers

- 1: process arrangement
- 2: aqueous-two-phase system
- 3: fluidized bed centrifuge
- 4: chamber
- 5: chamber inlet
- 6: chamber outlet
- 7: first phase
- 8: second phase
- 9: interphase
- 10: heavy phase
- 11: light phase
- 12: bioreactor
- 13: cell broth
- 14: two phase-forming components
- 15: phosphate salt
- 16: PEG
- 17: media vessel
- 18: waste vessel
- 19: static mixer
- 20: tube set
- 21: conductivity sensor
- 22: output fluid line
- 23: valve
- 24: exit valves
- 25: exit port
- 26: input fluid line
- 27: input port
- 28: elutriation boundary
- 29: priming port
- 30: media port
- 31: media pump
- 32: chamber pump
- 33: bypass valve
- 34: input switch valve
- 35: harvest vessel
- 36: harvest port
- 37: waste port
- 38: further processing
- 39: control unit

## Claims

1. Method of operating a process arrangement (1), in particular bioprocess arrangement, wherein the method comprises a separation of two phases of an aqueous-two-phase system (2),
**characterized in**
**that** the process arrangement (1) comprises a fluidized bed centrifuge (3), that the fluidized bed centrifuge (3) is used for the separation of the two phases of the aqueous-two-phase system (2), that the aqueous-two-phase system (2) is loaded into a rotating chamber (4) of the fluidized bed centrifuge (3) as a fluid flow, that due to the rotation of the chamber (4) the aqueous-two-phase system (2) is centrifuged such that a first phase (7) of the two phases is separated from a second phase (8) of the two phases, and, that due to the fluid flow during the rotation of the chamber (4), the first phase (7) remains in the chamber (4) while the second phase (8) leaves the chamber (4), such that the two phases are separated.

2. Method according to claim 1, **characterized in that** the bioprocess arrangement comprises a bioreactor (12), that a bioproduct is produced by a cell broth (13) in the bioreactor (12), that the cell broth (13) is transferred from the bioreactor (12) to the fluidized bed centrifuge (3), that the cell broth (13) is mixed with two phase-forming components (14), in particular phosphate salt (15) and polyethylene glycol (PEG (16)), to form the aqueous-two-phase system (2).

3. Method according to claim 2, **characterized in that** the bioproduct has a size of at most 5 µm, preferably at most 1 µm, more preferably at most 0,5 µm, and/or, that the bioproduct is a viral vector, preferably, that the bioproduct is an adenoviral vector or an adeno-associated viral vector or a lentiviral vector or a virus-like particle or a recombinant protein or a nucleic acid or a monoclonal antibody or an antibody-drug conjugate.

4. Method according to claim 2 or 3, **characterized in that** at least one of the two phase-forming components (14), preferably both phase-forming components, are added to the cell broth (13) inline while the cell broth (13) is flowing between the bioreactor (12) and the fluidized bed centrifuge (3), preferably, that the cell broth (13) and the two phase-forming components (14) are mixed inline by a static mixer (19), and/or, that after the two phase-forming components (14) have been added to the cell broth (13) thereby creating the aqueous-two-phase system (2), the aqueous-two-phase system (2) is transferred into the chamber (4) of the fluidized bed centrifuge (3) after at least 2 seconds, preferably at least 5 seconds, and/or at most 60 minutes, preferably at most 10 minutes, more preferably at most 2 minutes.

5. Method according to one of the preceding claims, **characterized in that** inside the centrifuge chamber (4) a light phase (11), in particular a PEG-rich phase, an interphase (9), in particular containing biomass, and a heavy phase (10), in particular a salt-rich phase, of the aqueous-two-phase system (2) are located at different regions of the chamber (4), preferably, that two of the three phases are retained in the chamber (4) and the third phase is flushed out of the chamber (4).

6. Method according to one of the preceding claims, **characterized in that** a tube set (20) preferably comprising a conductivity sensor (21) is connected to, in particular forms a single-use unit with, the fluidized bed chamber (4), preferably, that the conductivity sensor (21) is connected to an output fluid line (22) of the fluidized bed chamber (4), more preferably, that the conductivity sensor (21) is used to separate the two phases by adapting the fluid flow and/or by switching a valve (23) of the tube set (20) connected to the output fluid line (22).

7. Method according to one of the preceding claims, **characterized in that** a cycle of the fluidized bed centrifuge (3) comprises the following steps in this order:
a) the chamber (4) is loaded with the aqueous-two-phase system (2) as the fluid flow in the forward direction and the light phase (11) is flushed out of the chamber (4) in the forward direction during the loading while the heavy phase (10) remains in the chamber (4),
b) the loading of step a) is finished, preferably at a time determined based on an optical detection of a predefined loading state of the chamber (4),
c) the heavy phase (10) is flushed out of the chamber (4) in the backward direction, preferably,
that the cycle of the fluidized bed centrifuge (3) comprises before step a) a step of priming the chamber (4) through a priming port (29), more preferably, that the priming is done with the aqueous-two-phase system (2) or a priming liquid.

8. Method according to one of the preceding claims, **characterized in that** a cycle of the fluidized bed centrifuge (3) comprises the following steps in this order:
d) the chamber (4) is loaded with the aqueous-two-phase system (2) as the fluid flow in the backward direction and the heavy phase (10) is flushed out of the chamber (4) in the backward direction during the loading while the light phase (11) remains in the chamber (4),
e) the loading of step d) is finished, preferably at a time determined based on a detection of a predefined loading state of the chamber (4) by the conductivity sensor (21),
f) the light phase (11) is flushed out of the chamber (4) in the forward direction.

9. Method according to one of the preceding claims, **characterized in that** one or more conductivity sensors (21) are connected to the chamber inlet (5) and/or to the chamber outlet (6) to detect a predefined loading state of the chamber (4), and/or, that an optical sensor is used to detect the predefined loading state of the chamber (4) while the fluid flow is active in the forward and/or backward direction.

10. Method according to one of claims 7 to 9, **characterized in that** the bioproduct is located in the light phase (11), that in step a) the interphase (9) remains in the chamber (4) and in step c) the interphase (9) is flushed out of the chamber (4), and/or, that in step d) the interphase (9) is flushed out of the chamber (4).

11. Method according to claim 7 to 9, **characterized in that** the bioproduct is located in the heavy phase (10), that in step a) the interphase (9) is flushed out of the chamber (4), and/or, that in step d) the interphase (9) remains in the chamber (4) and in step f) the interphase (9) is flushed out of the chamber (4).

12. Method according to one of claims 7 to 11, **characterized in that** the cycle of the fluidized bed centrifuge (3) comprises between steps b) and c) and/or steps e) and f) a step of further loading the chamber (4) with a replacement liquid as the fluid low thereby flushing bioproduct out of the tube set (20) behind the chamber (4).

13. Method according to one of claims 7 to 12, **characterized in that** an exit port (25) of the tube set (20) is connected to the vessel, in particular the bioreactor (12), containing the aqueous-two-phase system (2) and the heavy phase (10) and preferably the interphase (9) in step c) and/or the light phase (11) and preferably the interphase (9) in step f) is or are discharged into the bioreactor (12), and/or
that an exit port (25) of the tube set (20) is connected to a vessel, in particular the bioreactor (12), containing the aqueous-two-phase system (2) and the light phase (11) in step a) and/or heavy phase (10) in step d) is discharged into the bioreactor (12),
and/or,
that the media port (30) is connected to the vessel, in particular the bioreactor (12), containing the aqueous-two-phase system (2).

14. Method according to one of the preceding claims, **characterized in that** one or both phase-forming components are added to the bioreactor (12) or a subsequent vessel, that the cell broth (13) is transferred in batches to the fluidized bed centrifuge (3), that, for processing each batch, the phase containing the bioproduct is separated in the fluidized bed chamber (4) from the interphase (9) and the other phase, the phase containing the bioproduct is transferred to a further processing (38), preferably, that the other phase and the interphase (9) are returned to the bioreactor (12) or the vessel.

15. Method according to claim 14, **characterized in that** when the relative volume of one phase in the vessel, in particular bioreactor (12), fulfills a criterion, in particular reaches a threshold, the phase-forming component forming the phase containing the bioproduct is replenished in the vessel and further batches are processed, preferably, that the replenishment is done continuously with a flow rate equal to an average transfer of volume to the further processing (38).

16. Method according to claim 14 or 15, **characterized in that** depending on the relative volume of one phase in the bioreactor (12) or vessel a chamber (4) filling volume of the fluidized bed centrifuge (3) is adapted between batches.

17. Method according to one of claims 2 to 16, **characterized in that** the bioproduct is produced inside the cells, that at least one of the phase-forming components lyses the cells to release the bioproduct, preferably, that the cell broth (13) is mixed with a first of the two phase-forming components (14), in particular a salt-rich phase-forming component, preferably a phosphate salt (15), then the cells are lysed and then the cell broth (13) is mixed with the second of the two phase-forming components (14), or, that the cell broth (13) is mixed with both of the phase-forming components and that the combination of both phase-forming components lyses the cells.

18. Method according to one of claims 2 to 12 or 17, **characterized in that** a fraction of the cell broth (13) in the bioreactor (12) is transferred to the fluidized bed centrifuge (3), that the two phase-forming components (14) are added to the fraction of the cell broth (13) inline, that the cells are lysed, that the bioproduct is an intracellular bioproduct, in particular a viral vector, that the phase containing the bioproduct is separated in the fluidized bed chamber (4) from the interphase (9) and the other phase, that a fraction of the cell broth (13) remaining in the bioreactor (12) is further cultivated.

19. Tube set for use in the method according to one of claims 6 to 18, wherein the tube set (20) comprises a conductivity sensor (21), preferably, wherein the tube set (20) is a single-use tube set (20).

20. Process arrangement adapted to be used in the method according to one of claims 1 to 18.
